# EUROPEAN PATENT APPLICATION

(11) **EP 4 344 665 A1**
(43) Date of publication of application: **03.04.2024**
(21) Application number: 22205614.5
(22) Date of filing: 04.11.2022
(51) Int. Cl.: A61B 18/24, A61B 17/00, A61B 18/22

(54) **LASER CATHETER PROXIMAL COUPLER WITH CAPILLARY TUBE ASSEMBLY**

(30) Priority: 29.09.2022 US 202263411320 P
(71) Applicant: Koninklijke Philips N.V., 5656 AG Eindhoven (NL)
(72) Inventor: FORD, Kylie Cyrielle, Eindhoven (NL); CARVER, Robert, Eindhoven (NL); HARTLEY, Alexandra, 5656AG Eindhoven (NL); GELSINGER, Jacob James, 5656AG Eindhoven (NL)
(74) Representative: Philips Intellectual Property & Standards

(57) **Abstract**

A laser catheter assembly (10) includes a laser catheter (12) including a bundle of optical fibers (14); and a coupler (16) attached to a proximal end of the laser catheter. The coupler includes a connector (18) configured to mate with an associated light source; and a holding member (22) secured within the connector and having a through hole (24) positioned at an end of the channel, an end of the proximal portion of the bundle of optical fibers being disposed in the through hole, the through hole having a light input end (26) arranged to receive light from the associated light source when the connector is mated with the associated light source and a flared end (28) opposite the light input end.

## Description

### FIELD

The following relates generally to the catheter arts, vascular therapy, lesion treatment arts, and related arts.

### BACKGROUND

In catheter-based vascular therapy, a laser catheter utilizes 308 nm laser light transmitted down a catheter to the distal tip where the light can ablate tissue. The laser light can be used with a contrast mixture. The laser light breaks down the contrast and, in the process, generates a sonic wave. This sonic wave emanates in a circular or spherical wave around the tip of the laser catheter. When emitted in a calcified lesion, the sonic wave can create fractures in the calcium. However, because of the circular nature of the wave, if there is a specific section that does not have a calcified lesion that fully encapsulates the vessel, then a large portion of the generated energy from the pressure wave may bypass the targeted calcium.

Current laser catheter devices include a proximal coupler to align optical fibers within the catheter with the laser system so that the laser energy can be translated to the tip of the catheter for treatment. Current laser catheter devices can also include a capillary (or 'cap') tube and capillary tube clamp components of the assembly. The capillary tube is sized to absorb the laser beam and made from materials that withstand the laser energy to protect the adjacent coupler components. Treatments to the surface of the capillary tube can be applied to prevent transmission that may damage the adjacent components within the coupler.

Current laser catheter capillary tube devices can be produced from fused silica with an abraded surface that prevents transmission via light dispersion at the abraded surface. The channel within the capillary tube is used to align fibers with the laser beam. In addition, the clamp for the capillary tube is currently made of stainless steel and is used to hold the capillary tube in place within the proximal coupler assembly.

The following discloses certain improvements.

### SUMMARY

In some embodiments disclosed herein, a laser device assembly includes an elongate device, such as for example a laser catheter, including a bundle of optical fibers; and a coupler attached to a proximal end of the elongate device. The coupler includes a connector configured to mate with an associated light source; and a holding member secured within the connector and having a through hole positioned at an end of the channel, an end of the proximal portion of the bundle of optical fibers being disposed in the through hole, the through hole having a light input end arranged to receive light from the associated light source when the connector is mated with the associated light source and a flared end opposite the light input end.

In some embodiments disclosed herein, a method of assembling a laser catheter assembly includes providing a slide piece having a channel formed therein; securing a holding member to the slide piece with a through hole of the holding member aligned with the channel, the through hole having a flared end; disposing a proximal portion of a bundle of optical fibers of a laser catheter in the channel; sliding an end of the proximal portion of the bundle of optical fibers through the flared end of the through hole of the holding member such that the end of the proximal portion of the bundle of optical fibers is compressed into the through hole; and assembling first and second cover pieces of a connector around onto the slide piece to form a coupler attached to a proximal end of the laser catheter.

In some embodiments disclosed herein, a laser catheter assembly includes a laser catheter including a bundle of optical fibers; and a coupler attached to a proximal end of the laser catheter. The coupler includes a connector configured to mate with an associated light source; and a plate or disk secured within the connector and having a through hole positioned at an end of the channel, an end of the proximal portion of the bundle of optical fibers being disposed in the through hole.

One advantage resides in providing a flared end on a capillary tube in a laser catheter assembly to hold optical fibers in place.

Another advantage resides in using a disc instead of a capillary tube to hold optical fibers in place.

Another advantage resides in using a plate instead of a capillary tube to hold optical fibers in place.

Another advantage resides in using a malleable clamp to hold optical fibers in place.

A given embodiment may provide none, one, two, more, or all of the foregoing advantages, and/or may provide other advantages as will become apparent to one of ordinary skill in the art upon reading and understanding the present disclosure.

### BRIEF DESCRIPTION OF THE DRAWINGS

The disclosure may take form in various components and arrangements of components, and in various steps and arrangements of steps. The drawings are only for purposes of illustrating the preferred embodiments and are not to be construed as limiting the disclosure.
FIGURE 1 diagrammatically illustrates a laser catheter assembly in accordance with the present disclosure.
FIGURES 2-6 show embodiments of a holding member of the assembly of FIGURE 1.
FIGURES 7, 8A, 8B show embodiments of a clamp of the assembly of FIGURE 1.
FIGURE 9 shows a slide piece of the assembly of FIGURE 1.
FIGURE 10 diagrammatically illustrates a method of assembling the assembly of FIGURE 1.

### DETAILED DESCRIPTION

The following relates to improvements to a proximal-end coupler of a laser catheter for coupling the laser catheter with an excimer laser or other type of laser used in laser catheter procedures. The following presents improvements on the basic coupler which is described in Harlan et al., U.S. Pat. No. 7,050,692 (which is incorporated in its entirety by reference herein). The basic design has a slide mount sized and shaped to connect with the laser, with a channel that receives the optical fibers and positions the fiber ends at the laser output aperture, which is a rectangular aperture in commercial lasers in use currently. A clamp holds the optical fibers in the channel without the user of an adhesive. A capillary tube is placed at the end of the channel, and the ends of the optical fibers fit into the lumen of the capillary tube to provide a tightly packed bundle of optical fiber ends for coupling with the laser aperture.

In the present assembly sequence, the ends of the fibers are secured within the capillary tube, and that assembly is then placed into the channel of a bottom slide piece and secured with the clamp, and then the top slide piece is secured to complete the assembly.

In one improvement, the capillary tube has a chamfer that widens the end into which the optical fiber ends are inserted. Additionally, the outer diameter of the chamfer places it outside of the perimeter of the slide channel. With these modifications, the assembly process can be modified by first mounting and securing the capillary tube in the channel and then laying the optical fibers into the channel and sliding the optical fiber ends into the chamfered capillary tube.

In another improvement, the capillary tube may be replaced by a larger assembly, such as a plate or disk, which has an opening with an inner diameter that serves the same purpose as the inner diameter of the capillary tube, i. e. to form the fiber ends into a tight bundle for coupling with the laser aperture. This approach has advantages in terms of manufacturability as the plate or disk can be a stock plate that is machined to form the capillary opening, as opposed to the extrusion process used in the capillary tube.

In another improvement, the capillary opening is replaced by a rectangular opening that better matches a typically rectangular laser beam aspect ratio. This improvement is facilitated by using the plate or disk as the machining of the stock plate can more easily form a rectangular opening; however, it may also be implemented as a capillary tube with suitable extrusion techniques.

In another improvement, the existing metal clamp is replaced by a clamp of a malleable material such as plastic or rubber, which is less likely to damage the capillary tube (or the replacement plate or disk). In variant embodiments, the clamp is replaced by malleable material disposed on the top slide piece so as to eliminate the need for the clamp fasteners.

In a further variant, the malleable clamp has a cutout or notch, referred to herein as a window, to enhance visibility during the assembly of the proximal coupler.

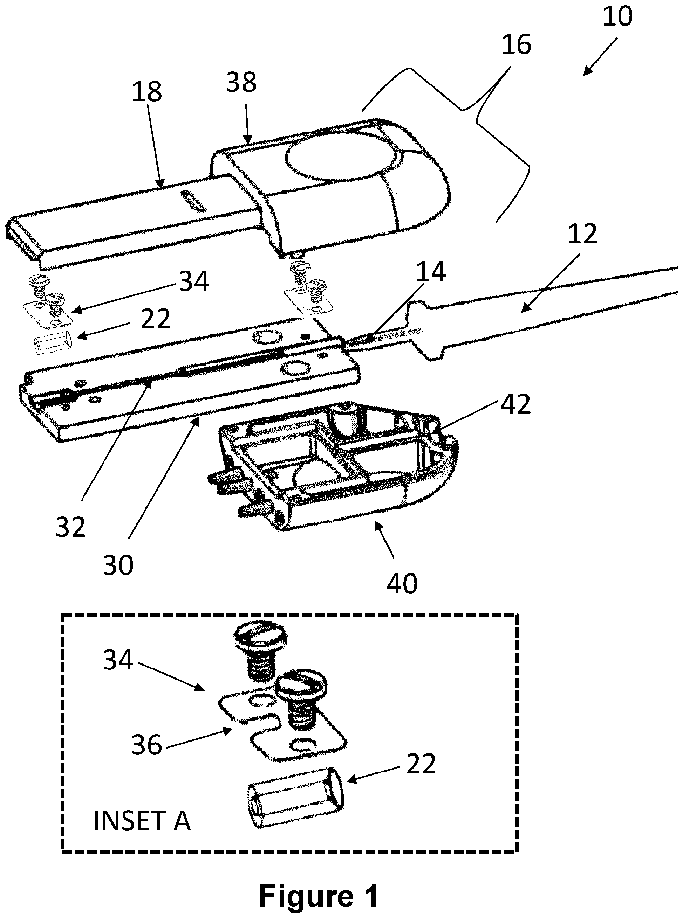

With reference to FIGURE 1, an illustrative laser catheter assembly 10 insertable is diagrammatically shown. As shown in FIGURE 1, the device 10 includes an elongate device 12, such as for example a laser catheter, including at least one optical fiber 14 (shown in FIGURE 1 as a bundle of optical fibers) extending through an elongate device 12. The laser catheter 12 may also comprise a strain relief portion thereof.

A coupler (generally shown in FIGURE 1 as element 16 in FIGURE 1) is attached to a proximal end of the laser catheter 12. As shown in FIGURE 1, the coupler 16 includes a connector 18 configured to mate, or otherwise attached, with an associated light source (not shown in FIGURE 1) configured to supply laser light to the optical fibers 14. A portion of the bundle of optical fibers 14 is disposed within the connector 18.

The coupler 16 also includes a holding member 22 secured within the connector 18. As best shown in INSET A of FIGURE 1, the holding member 22 includes a through hole 24 through which end of the proximal portion of the bundle of optical fibers 14 can be in the through hole 24 of the holding member 22.

FIGURE 1 also shows that the coupler 16 includes a slide piece 30 configured to mate with the light source. The slide piece 30 includes a channel 32 formed therein that is configured to receive and secure the bundle of fibers 14 within the slide piece 30. The slide piece 30 is configured to connect with the connector 18 to secure the holding member 22 and the bundle of optical fibers 14 therebetween.

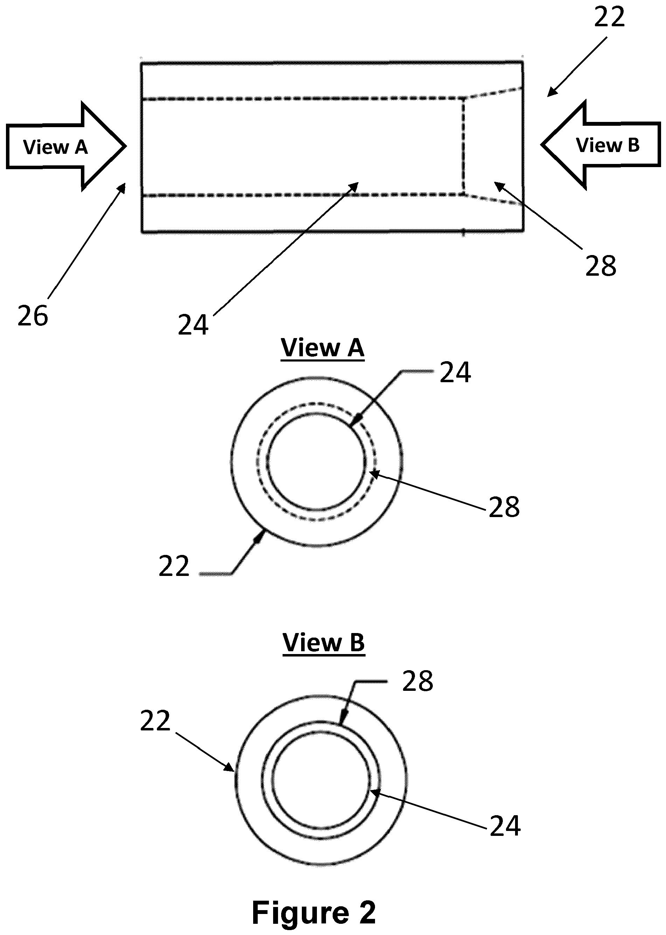

With continuing reference to FIGURE 1, FIGURE 2 shows an embodiment of the holding member 22. As shown in FIGURE 2, the holding member 22 comprises a capillary tube having a light input end 26 arranged to receive light from the light source when the slide piece 30 is mated with the light source, and a flared end 28 opposite the light input end 26. As shown in FIGURE 2, the through hole 24 comprises a lumen of the capillary tube 22 having the flared end 28.

In some embodiments, as shown in View A of FIGURE 2, an outer diameter of the flared end 28 of the through hole 24 is larger than a perimeter of the channel 32 of the slide piece 30. This allows the bundle of optical fibers 14 to run from through the through hole 24 to the laser catheter 12 and reducing chances of damage to the optical fibers 14. View B of FIGURE 2 shows an opposing view of the holding member 22.

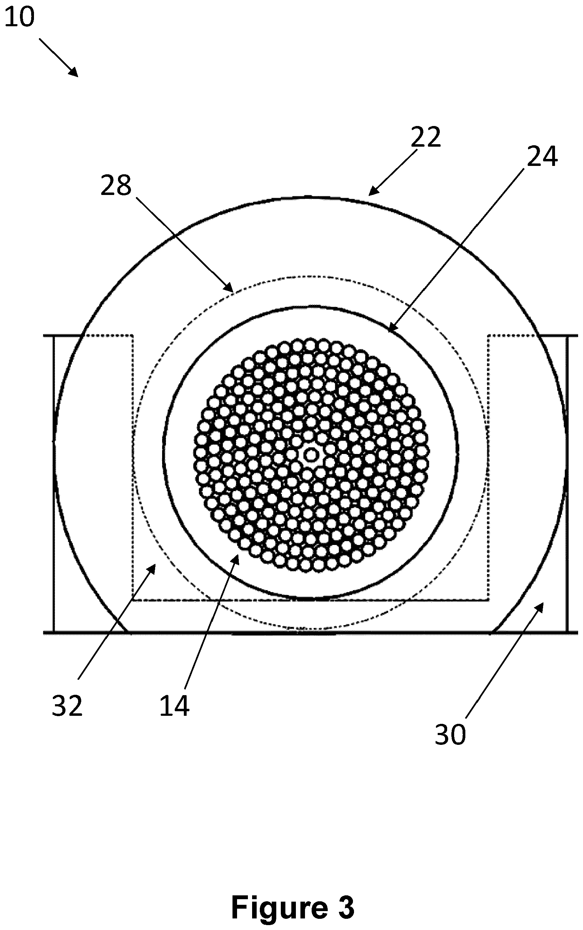

Referring now to FIGURE 3, a bottom portion of the inner diameter of the capillary tube 22 is also aligned with the bottom of the channel 32 of the slide piece 30 to reduce bending that might break or misalign the optical fibers 14 that could cause breakage and to aid in manufacturability. This allows the optical fibers 14 to slide along the channel 32 and right into the tapered end 28 of the through hole 24.

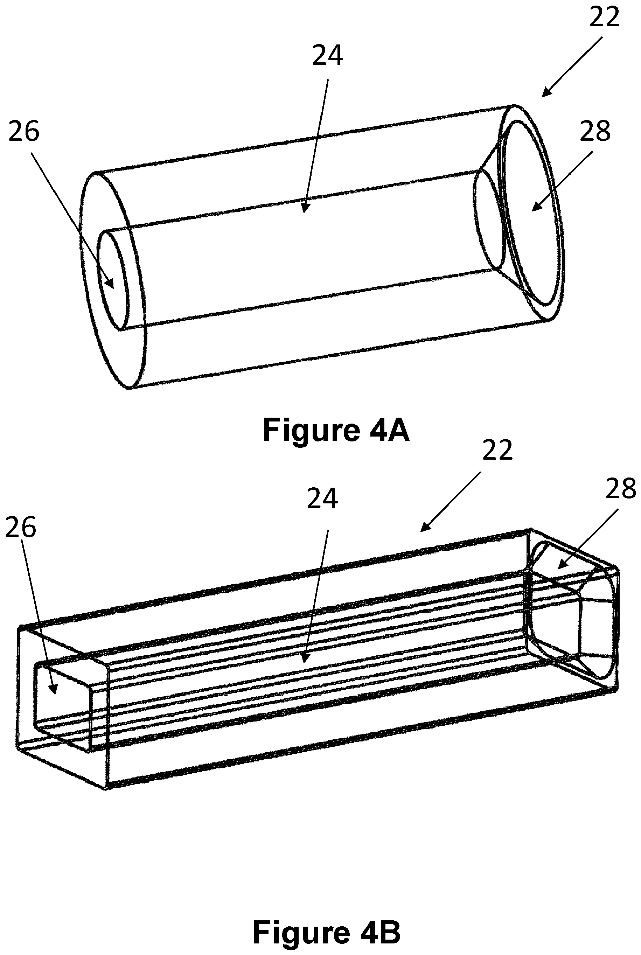

With continuing reference to FIGURES 1 and 2, FIGURES 4A and 4B show different embodiments of the capillary tube 22. As shown in FIGURE 4A, the through hole 24 has a circular cross-section, while in FIGURE 4B, the through hole 24 has a rectangular cross-section. FIGURE 4A also shows the tapered end 28 of the through hole 24.

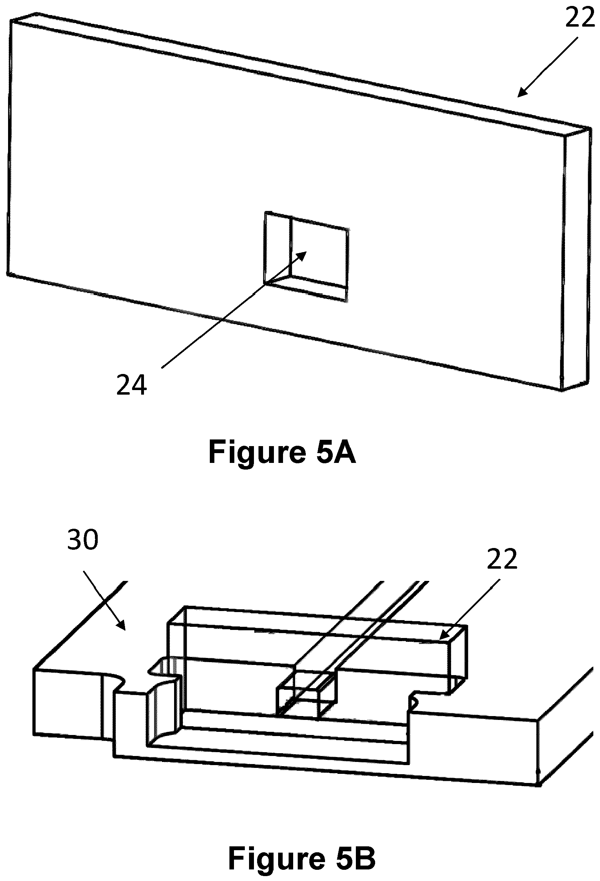

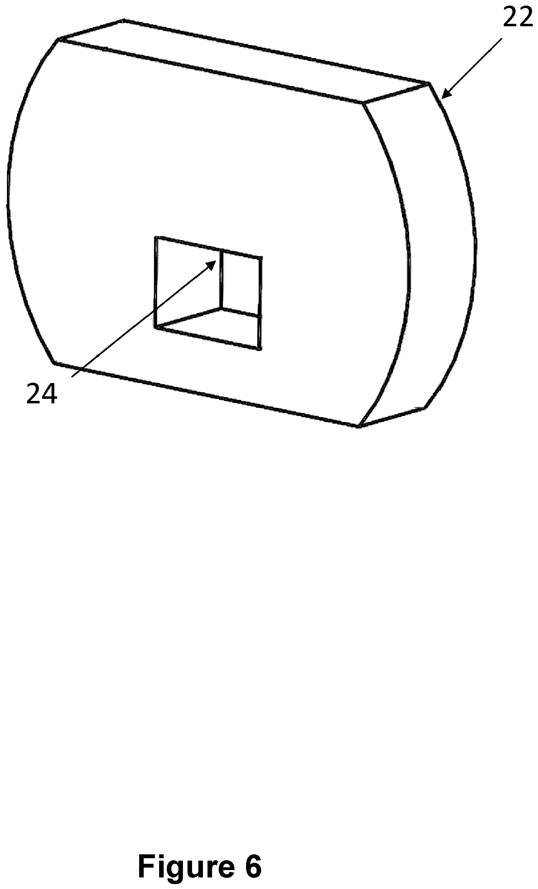

FIGURES 5A, 5B, and 6 show other embodiments of the holding member 22. As shown in FIGURES 5A and 5B, the holding member 22 comprises a plate 22 including the through hole 24 passing therethrough. FIGURE 5B shows the plate 22 positioned within the slide piece 30. FIGURE 6 shows the holding member 22 comprising a disk 22 including the through hole 24 passing therethrough. The plate/disk 22 can provide more flexibility in fabricating sizes and shapes of the through hole 24 that match the shape and size of the laser beam profile supplied by the light source. There are possible cost reductions with thinner materials that are compatible with various machining methods. For example, the plate/disk 22 can be water jet or diamond cut instead of the drawing process that is limited to cylinders. The plate/disk 22 shape can also be tailored to the slide piece 30 to enable greater coverage to protect the adjacent components from laser energy.

Referring back to FIGURE 1, the laser catheter apparatus 10 further includes a clamp 34 configured to secure the holding member 22 to the slide piece 30 with the through hole 24 positioned at the end of the channel 32 of the slide piece 30. The clamp 34 can be secured to the slide piece 30 (for example, with screws as shown in FIGURE 1). The clamp 34 can comprise a malleable material, such as for example plastic and/or rubber. The malleable material of the clamp 34 reduces a likelihood of damage to the holding member 22. The clamp 34 can be positioned over the capillary tube 22 to prevent motion thereof. In some examples, the clamp 34 can be sized to allow visualization of the capillary tube 22 for a user during assembly of the laser catheter assembly 10. In other examples, as shown in INSET A of FIGURE 1, the clamp 34 can include notch 36 to enhance visibility during the assembly.

FIGURE 1 also shows that the connector 18 further includes a first cover piece 38 (i.e., a "top" cover piece as shown in FIGURE 1) and a second cover piece 40 (i.e., a "bottom" cover piece as shown in FIGURE 1). The slide piece 30 is secured between the first cover piece 38 and the second cover piece 40. The clamp 34 is secured to the slide piece 30 (i.e., with screws, as shown in FIGURE 1). In some embodiments, the clamp 34 can be secured to the first cover piece 38 or to the second cover piece 40. The second cover piece 40 includes a slot 42 configured to receive and secure the laser catheter 12. In some embodiment the slot 42 can be included in the first cover piece or in both, the first and second cover pieces. In some examples, the connector 18 and the first side piece 38 or first cover piece can be a single component.

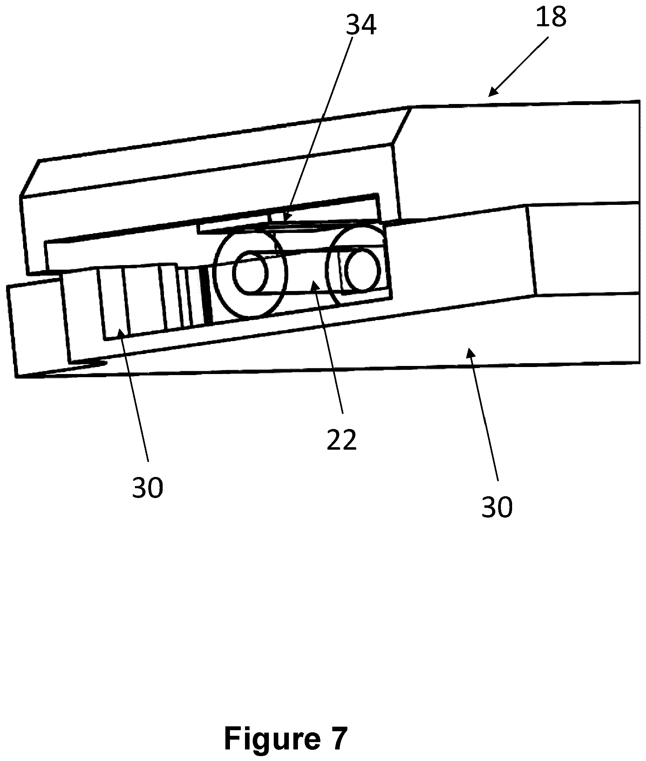

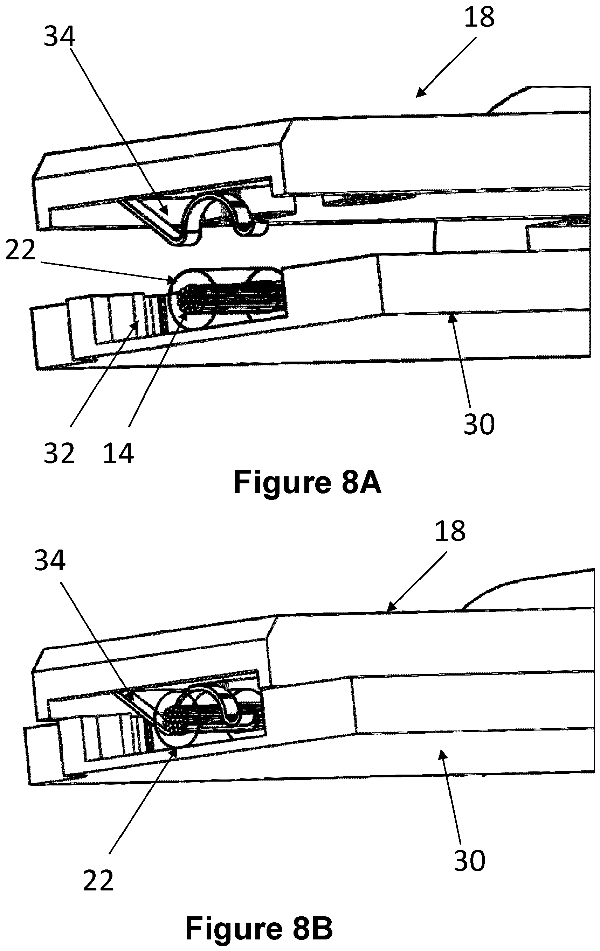

With continuing reference to FIGURE 1, FIGURES 7, 8A, and 8B show different embodiments of the clamp 34. As shown in FIGURE 7, the clamp 34 is secured to a portion of the first cover piece 38. The clamp 34 is configured to apply a compressive force to the capillary tube 22 to hold the capillary tube 22 in place in the channel 32 of the slide piece 30. FIGURES 8A and 8B show an embodiment where the clamp 34 comprises a spring configured to engage and hold the capillary tube 22 in place in the channel 32 of the slide piece 30. FIGURE 8A shows the clamp 34 prior to engagement with the capillary tube 22, and FIGURE 8B shows the clamp 34 engaged with the capillary tube 22.

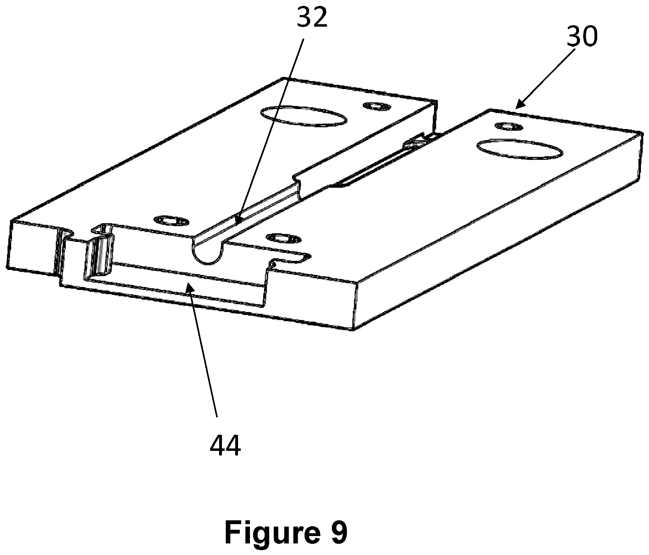

FIGURE 9 shows a view of the slide piece 30. As shown in FIGURE 9, the slide piece 30 includes the channel 32 for receiving the bundle of optical fibers 14. The slide piece 30 can also include a recess 44 configured to receive the holding member 22 (i.e., either the capillary tube 22 or the plate/disk 22). The recess 44 can be machined into the slide piece 30 at the desired setback based on the size of the laser catheter 12 and a number of optical fibers 14. The laser beam profile becomes larger at larger setbacks, therefore smaller fiber packs have a smaller setback (i.e., closer to the edge of the slide piece 30) and larger fiber packs have a larger setback (farther from the edge of the slide piece 30). In some examples, the recess 44 can have a "dog bone" shape, a V-channel or similar structure that holds the capillary tube 22 in place. The recess 44 is machined in a way that allows the capillary tube 22 to align with the channel 32 of the slide piece 30 to be more manufacturable and reduce the risk of fiber breakage. To keep the capillary tube 22 within the feature, the clamp 34 is placed on top of the capillary tube 22.

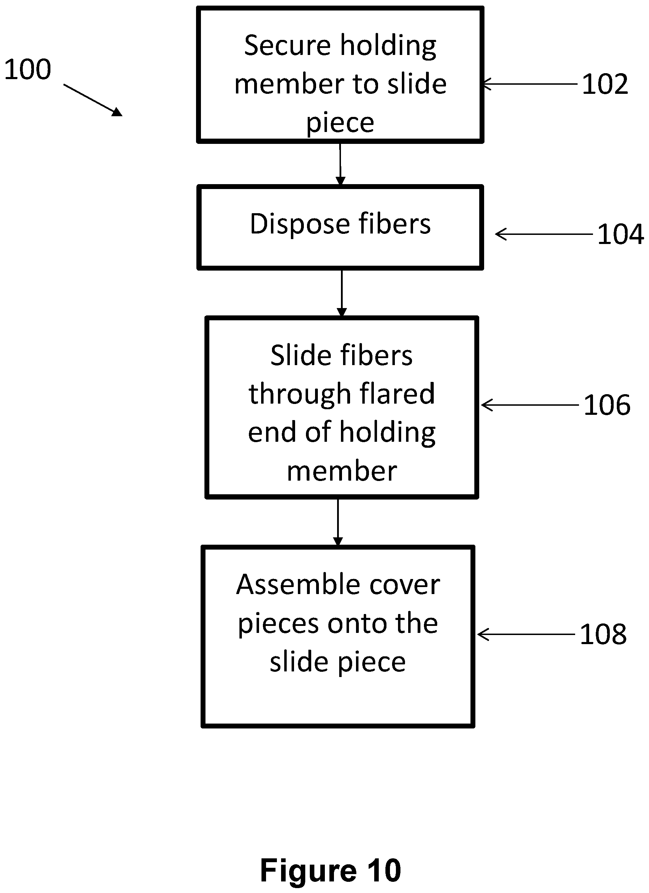

Referring to FIGURE 10, an illustrative embodiment of a method or process 100 of assembling the laser catheter assembly 10 is diagrammatically shown as a flowchart. At an operation 102, the holding member 22 is secured to the slide piece 30 so that the through hole 24 of the holding member 22 is aligned with the channel 32 of the slide piece 30. In some embodiments, the securing operation 102 can comprise clamping the holding member 22 to the slide piece 30 with the clamp 34.

At an operation 104, a proximal portion of the bundle of optical fibers 14 is disposed within the channel 32 of the slide piece 30. At an operation 106, an end of the proximal portion of the bundle of optical fibers 14 is slid through the flared end 28 of the through hole 24 of the holding member 22 so that the end of the proximal portion of the bundle of optical fibers 14 is compressed into the through hole 24. The outer diameter of the flared end 28 of the through hole 24 is larger than a perimeter of the channel 32 of the slide piece 30. During the sliding operation 106, the flared end 28 guides the end of the proximal portion of the bundle of optical fibers 14 into the through hole 24.

At an operation 108, the first cover piece 38 and the second cover piece 40 are assembled on opposing ends of the slide piece 30 to form the coupler 16. In some embodiments, the securing operation 102 is performed during the assembling operation 108 by securing the clamp 34 to the first cover piece 38, and the clamp 34 presses the holding member 22 against the slide piece 30 during the assembling operation 108. Once formed, the coupler 16 can be attached to the laser source, and a laser beam can be supplied to the optical fibers 14.

The disclosure has been described with reference to the preferred embodiments. Modifications and alterations may occur to others upon reading and understanding the preceding detailed description. It is intended that the exemplary embodiment be construed as including all such modifications and alterations insofar as they come within the scope of the appended claims or the equivalents thereof.

## Claims

1. A laser device assembly (10), comprising:
an elongate device (12) including a bundle of optical fibers (14); and
a coupler (16) attached to a proximal end of the elongate device, the coupler including:
a connector (18) configured to mate with an associated light source; and
a holding member (22) secured within the connector and having a through hole (24), an end of a proximal portion of the bundle of optical fibers being disposed in the through hole, the through hole having a light input end (26) arranged to receive light from the associated light source when the connector is mated with the associated light source and a flared end (28) opposite the light input end.

2. The laser device assembly (10) of claim 1, wherein the holding member (22) comprises a capillary tube, and the through hole (24) comprises a lumen of the capillary tube having the flared end.

3. The laser device assembly (10) of claim 1 or 2, wherein the through hole (24) of the holding member (22) has a rectangular cross-section.

4. The laser device assembly (10) of claim 1 or 3, wherein the holding member (22) comprises a plate or a disk having the through hole (24) passing through the plate or disk.

5. The laser device assembly (10) of any one of claims 1 to 4, wherein the connector (18) of the coupler (16) comprises a slide piece (30) configured to mate with the associated light source, the slide piece having a channel (32) formed therein, the coupler further including:
a clamp (34) securing the holding member to the slide piece with the through hole (24) positioned at the end of the channel.

6. The laser device assembly (10) of claim 5, wherein an outer diameter of the flared end (28) of the through hole (24) is larger than a perimeter of the channel (32) of the slide piece (30).

7. The laser device assembly (10) of claim 5 or 6, wherein the clamp (34) comprises a plastic or rubber.

8. The laser device assembly (10) of any one of claims 5 to 7, wherein the clamp (34) includes a notch (36).

9. The laser device assembly (10) of any one of claims 5 to 8, wherein the connector (18) further includes a first cover piece (38) and a second cover piece (40), the slide piece (30) being secured between the first and second cover pieces.

10. The laser device assembly (10) of claim 9, wherein the clamp (34) is secured to the first cover piece.

11. The laser device assembly (10) of claim 9 or 10, wherein the clamp (34) is secured to the slide piece.

12. A method (100) of assembling a laser device assembly (10), the method comprising:
providing a slide piece (30) having a channel (32) formed therein;
securing a holding member (22) to the slide piece with a through hole (24) of the holding member aligned with the channel, the through hole having a flared end (28);
disposing a proximal portion of a bundle of optical fibers (14) of an elongate device (12) in the channel;
sliding an end of a proximal portion of the bundle of optical fibers through the flared end of the through hole of the holding member such that the end of the proximal portion of the bundle of optical fibers is compressed into the through hole; and
assembling first and second cover pieces (38, 40) of a connector (18) around the slide piece to form a coupler (16) attached to a proximal end of the elongate device.

13. The method (100) of claim 12, wherein an outer diameter of the flared end (28) of the through hole (24) is larger than a perimeter of the channel (32) of the slide piece (30), whereby during the sliding the flared end guides the end of the proximal portion of the bundle of optical fibers (14) into the through hole of the holding member (22).

14. The method (100) of claim 12 or 13, wherein the securing of the holding member (22) to the slide piece (30) is performed during the assembling of the first and second cover pieces (38, 40) by a clamp (34) secured to the first cover piece that presses the holding member (22) against the slide piece during the assembling.

15. The method (100) of claim 12 or 13, wherein the securing of the holding member (22) to the slide piece (30) comprises clamping the holding member to the slide piece using a clamp (34) comprising a plastic or rubber.
